# EUROPÄISCHE PATENTSCHRIFT

(11) **EP 4 252 706 B1**
(45) Veröffentlichungstag und Bekanntmachung des Hinweises auf die Patenterteilung: **20.08.2025**
(21) Anmeldenummer: 22165854.5
(22) Anmeldetag: 31.03.2022
(51) Int. Cl.: A61C 13/00, B33Y 10/00, B33Y 30/00, B33Y 50/00

(54) **AUTOMATISCHE ERKENNUNG DENTALER INDIKATIONEN**
AUTOMATIC DETECTION OF DENTAL INDICATIONS
DÉTECTION AUTOMATIQUE D'INDICATIONS DENTAIRES

(43) Veröffentlichungstag der Anmeldung: 04.10.2023
(73) Patentinhaber: Ivoclar Vivadent AG, 9494 Schaan (LI)
(72) Erfinder: FREY, Alen, 6800 Feldkirch (AT); ASTER, Marius, 39020 Kastelbell/ Tschars (IT); JOHN, Hendrik, 9470 Buchs (CH)
(74) Vertreter: Baldus, Oliver

(56) Entgegenhaltungen:
- EP-A1- 3 868 330
- EP-A1- 4 113 373
- US-A1- 2021 255 600

## Beschreibung

Die vorliegende Erfindung betrifft ein Erkennungsverfahren für ein Dentalobjekt, ein Herstellungsgerät für ein Dentalobjekt und ein Computerprogramm zum Erkennen eines Dentalobjekts.

In bekannten digitalen Arbeitsabläufen werden in der CAD-Software dentale Restaurationen erzeugt und in einem 3D-CAD-Dateiformat abgespeichert. In Abhängigkeit von der verwendeten CAD-Software und dem sich anschließenden Arbeitsablauf können zusammen mit der 3D-CAD-Datei sogenannte Metadaten erzeugt und abgelegt werden, die zusätzliche umfassen Informationen.

Metadaten können hilfreich sein, um die abtragenden Fertigungsprozesse zu steuern oder optimieren, wie beispielsweise Frässtrategien für eine speziellen Restaurationstyp zuordnen zu können. In dreidimensionalen Druckverfahren, bei denen Stützstrukturen erforderlich sind, wie beispielsweise in der Stereolithografie, einer VAT-Polymerisation, einem 3D-DLP-Druck, oder einem selektiven Laser-Sintern von Metallen, sollten bestimmte Flächen von dentalen Restaurationen frei von der Anbindung von Stützstrukturen sein, um eine Präzision und Flächenqualität positiv zu beeinflussen.

Bei Metadaten besteht jedoch der Nachteil, dass diese bereits zuvor in der CAD-Software erzeugt werden und es sich um proprietäre Datenformate handelt. In diesem Fall muss eine verwendete CAM-Software die zugeordneten proprietären Metadaten importieren und interpretieren können. Da diese Metadaten-Formate nicht universell sind, muss für jedes proprietäre Metadaten-Format eine eigene Schnittstelle programmiert werden. Liegen keine Metadaten zu einem Objekt vor, kann die CAM-Software beim Import nicht bestimmen, um welche Indikation es sich handelt.

Allerdings ist die Indikation wichtig, um zu bestimmen, mit welchem Herstellungsverfahren ein Dentalobjekt hergestellt werden soll. Die Klassifizierung kann zudem bei der weiteren Datenverarbeitung und für die Bestimmung von weiteren Prozessschritten zur Fertigung oder zur Materialverarbeitung im digitalen Arbeitsablauf verwendet werden.

Die Druckschrift US 10,856,957 B2 betrifft ein Verfahren zum Herstellen eines dreidimensionalen digitalen Modells einer Prothesenbasis zur Herstellung unter Verwendung einer auf Licht basierenden dreidimensionalen Druckvorrichtung.

Die Druckschriften US 2021/255,600 A1 und EP 3 868 330 A1 betreffen ein Verfahren zur Herstellung einer dentalen Restauration, ein Computerprogramm zur Herstellung einer dentalen Restauration und eine Fräsvorrichtung zum Herstellen einer dentalen Restauration. Zum automatischen Integrieren von räumlichen Daten für Haltestege in einen dreidimensionalen Datensatz eines Rohlings wird ein Machine-Learning-Algorithmus verwendet, der aus vorangehenden Beispielen in Form von dreidimensionalen Datensätzen lernt und diese nach Beendigung der Lernphase so verallgemeinern kann, dass Haltstege automatisch berechnet werden. Dazu kann dieser beim maschinellen Lernen ein statistisches Modell aufbauen, das auf Trainingsdaten beruht. Dabei werden Muster und Gesetzmäßigkeiten in den Trainingsdaten für die Positionierung von Haltestegen erkannt. Der Machine-Learning-Algorithmus kann beispielsweise ein angelerntes neuronales Netz umfassen.

Die Druckschrift EP 4 113 373 A1, publiziert am 01.04.2023, beansprucht das Prioritätsdatum 01.07.2021 und ist Stand der Technik gemäß Art. 54(3) EPÜ und betrifft computerimplementierte Methoden und Produkte zur Unterstützung zahnärztlicher Verfahren. Sie offenbart ein Verfahren zur Klassifizierung einer Zahnstruktur unter Verwendung einer trainierten datengesteuerten Logik, wobei das Verfahren das Bereitstellen eines 2D-Bildes als Eingabe für die trainierte datengesteuerte Logik und das Bestimmen eines Klassifizierungswertes des 2D-Bildes mittels der datengesteuerten Logik umfasst.

Die technische Aufgabe der Erfindung besteht darin, die Indikation von digitalen Dentalobjekten anhand der geometrischen Form zu erkennen, so dass ein passendes Herstellungsverfahren ausgewählt werden kann.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Erkennungsverfahren für ein Dentalobjekt gelöst, mit den Schritten eines Bereitstellens eines digitalen Dentalobjekts in einem Koordinatensystem, das eine Form des herzustellenden Dentalobjekts beschreibt; eines automatischen Zuordnens des digitalen Dentalobjektes zu einer vorgegebenen Klasse auf Basis der Form durch einen selbstlernenden Algorithmus; eines Zuordnen eines Herstellungsverfahrens zu dem digitalen Dentalobjekt auf Basis der zugeordneten Klasse; und eines Hinzufügens weiterer räumlicher Strukturen zu dem digitalen Dentalobjekt auf Basis des zugeordneten Herstellungsverfahrens. Der Klasse kann ein bestimmtes Herstellungsverfahren zugeordnet sein, mit dem sich das reale Dentalobjekt auf Basis des digitalen Dentalobjekts herstellen lässt. Je nach Klasse des Dentalobjekts können angepasste Herstellungsverfahren mit optimalen Parametern verwendet werden. Die räumlichen Strukturen können Stützstrukturen für eine Bauplattform oder Haltestrukturen für einen Rohling sein. Dadurch wird beispielsweise der technische Vorteil erreicht, dass sich die Herstellung des Dentalobjekts weiter verbessern lässt. Dadurch wird beispielsweise der technische Vorteil erreicht, dass sich das Dentalobjekt effizient herstellen lässt und sich die Herstellung unterschiedlicher Dentalobjekte automatisieren lässt.

In einer technisch vorteilhaften Ausführungsform des Erkennungsverfahrens wird eine Anzahl von Punkten auf der Oberfläche des digitalen Dentalobjekts erfasst. Dadurch wird beispielsweise der technische Vorteil erreicht, dass sich das digitalen Dentalobjekt mit einer geringen Menge an Klassifizierungsdaten schnell zuordnen lässt.

In einer weiteren technisch vorteilhaften Ausführungsform des Erkennungsverfahrens werden die Punkte auf der Oberfläche des digitalen Dentalobjekts zufällig ausgewählt. Dadurch wird beispielsweise der technische Vorteil erreicht, dass das die Grundgesamtheit an Klassifizierungsdaten ohne komplizierte Berechnungen gewonnen werden kann.

In einer weiteren technisch vorteilhaften Ausführungsform des Erkennungsverfahrens bilden die Koordinaten der erfassten Punkte eine Eingabe für ein künstliches neuronales Netz. Dadurch wird beispielsweise der technische Vorteil erreicht, dass sich das digitale Dentalobjekt effizient einer Klasse zuordnen lässt.

In einer weiteren technisch vorteilhaften Ausführungsform des Erkennungsverfahrens ist das künstliche neuronales Netz durch eine Mehrzahl von Trainingsdatensätzen angelernt worden. Dadurch wird beispielsweise ebenfalls der technische Vorteil erreicht, dass sich das digitale Dentalobjekt effizient einer Klasse zuordnen lässt.

In einer weiteren technisch vorteilhaften Ausführungsform des Erkennungsverfahrens wird die Klasse des digitalen Dentalobjekts durch das künstliche neuronale Netz ausgegeben. Dadurch wird beispielsweise ebenfalls der technische Vorteil erreicht, dass sich das digitale Dentalobjekt effizient einer Klasse zuordnen lässt.

In einer weiteren technisch vorteilhaften Ausführungsform des Erkennungsverfahrens wird dem digitalen Dentalobjekt auf Basis der zugeordneten Klasse ein digitales Referenzobjekt zugeordnet. Dadurch wird beispielsweise der technische Vorteil erreicht, dass ein Arbeitsablauf weiter optimiert werden kann.

In einer weiteren technisch vorteilhaften Ausführungsform des Erkennungsverfahrens wird das digitale Dentalobjekt auf Basis der zugeordneten Klasse und/oder des Referenzobjekts im Koordinatensystem transformiert. Das digitale Dentalobjekt kann beispielsweise an dem Referenzobjekt ausgerichtet werden oder zu dem Referenzobjekt verschoben werden. Dadurch wird beispielsweise der technische Vorteil erreicht, dass das Herstellungsverfahren mit einer angepassten Orientierung und/oder Position des digitalen Dentalobjekt durchgeführt werden kann.

In einer weiteren technisch vorteilhaften Ausführungsform des Erkennungsverfahrens wird das Dentalobjekt durch das Herstellungsverfahren hergestellt. Dadurch wird beispielsweise der technische Vorteil erreicht, dass das Dentalobjekt mit einem geeigneten Herstellungsverfahren hergestellt wird.

In einer weiteren technisch vorteilhaften Ausführungsform des Erkennungsverfahrens ist das Herstellungsverfahren ein additives oder subtraktives Herstellungsverfahren. Dadurch wird beispielsweise der technische Vorteil erreicht, dass besonders geeignete Herstellungsverfahren für Dentalobjekte verwendet werden.

In einer weiteren technisch vorteilhaften Ausführungsform des Erkennungsverfahrens wird eine Korrektheit der Zuordnung durch geometrische Merkmale des digitalen Dentalobjekts überprüft. Dadurch wird beispielsweise der technische Vorteil erreicht, dass sich die Genauigkeit des Verfahrens verbessert.

Gemäß einem zweiten Aspekt wird die technische Aufgabe durch ein Herstellungsgerät für ein Dentalobjekt gelöst, das ausgebildet ist, das Erkennungsverfahren nach dem ersten Aspekt durchzuführen. Durch das Herstellungsgerät werden die gleichen technische Vorteile wie durch das Erkennungsverfahren gelöst.

Gemäß einem dritten Aspekt wird die technische Aufgabe durch ein Computerprogramm gelöst, mit Befehlen, die bei der Ausführung des Computerprogramms durch einen Computer diesen veranlassen, das Verfahren nach dem ersten Aspekt auszuführen. Durch das Computerprogramm werden die gleichen technische Vorteile wie durch das Erkennungsverfahren gelöst.

Ausführungsbeispiele der Erfindung sind in den Zeichnungen dargestellt und werden im Folgenden näher beschrieben.

Es zeigen:
- Fig. 1: eine schematische Darstellung von Klassifikationsverfahren;
- Fig. 2: eine Vielzahl von Punkten aus einem Klassifikationsdatensatz;
- Fig. 3: eine schematische Ansicht eines künstlichen neuronalen Netzes;
- Fig. 4: digitale Dentalobjekte unterschiedlicher Klassen;
- Fig. 5: eine Anzeige einer erkannten Klasse; und
- Fig. 6: ein Blockdiagramm eines Erkennungsverfahren für ein Dentalobjekt.

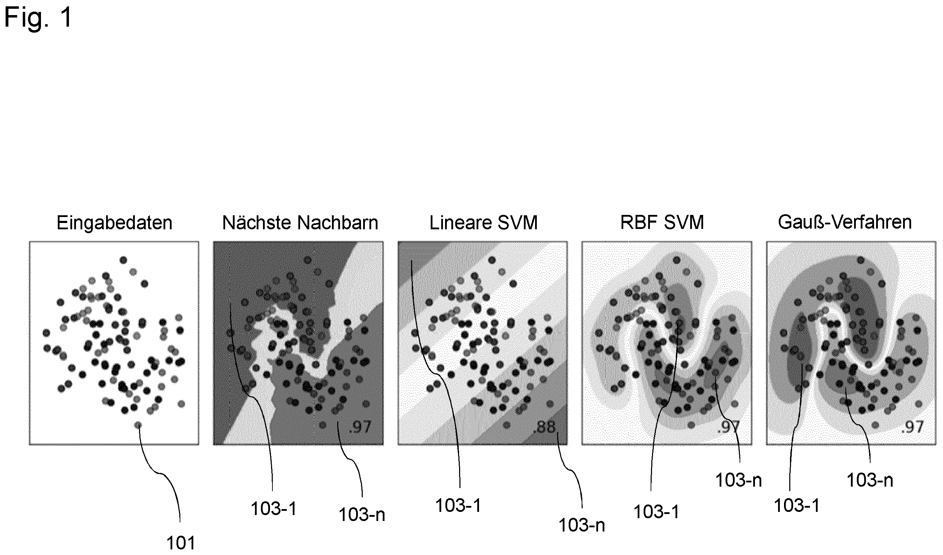

Fig. 1 zeigt eine schematische Darstellung von Klassifikationsverfahren. Ein reales Dentalobjekt 100-2 ist beispielsweise eine Krone, eine Brücke, ein Veneer, ein Abutment, ein Inlay, ein Onlay, eine Schiene oder eine Teil- oder Vollprothese. Im Allgemeinen kann das Dentalobjekt 100-2 jedes Objekt im Dentalbereich sein, das im Rahmen eines dreidimensionalen Herstellungsverfahrens additiv oder subtraktiv hergestellt werden soll.

Zu jedem dieser realen Dentalobjekte 100-2 kann ein digitales Dentalobjekt 100-1 existieren, in dem die dreidimensionale Form und die Farbwerte angegeben sind. Diese Informationen sind beispielswese in einem Datensatz zu dem digitalen Dentalobjekt 100-1 gespeichert.

Mithilfe einer Klassifizierung des digitalen Dentalobjektes 100-1 kann festgestellt werden, zu welchem Typ dieses gehört. Der Typ des digitalen Dentalobjektes 100-1 kann beispielsweise "Krone", "Brücke", "Veneer", "Abutment", "Inlay", "Onlay", "Schiene" oder "Teilprothese" oder "Vollprothese" sein. Im Allgemeinen kann der Typ des digitalen Dentalobjektes 100-1 jeder Typ sein, anhand dessen sich reale Dentalobjekte 100-2 voneinander unterscheiden lassen.

Eine Unterscheidung der digitalen Dentalobjekte 100-1 kann beispielsweise auf Basis der jeweiligen Form durchgeführt werden. Die Klassifikation kann daher auf Basis von Klassifikationsdatensätzen 101 durchgeführt werden, die eine Vielzahl von Punkten auf der Oberfläche des digitalen Dentalobjektes 100-1 umfassen. Sind zahlreiche Punkte 115 auf der Oberfläche des digitalen Dentalobjektes 100-1 bekannt, kann beispielsweise automatisch festgestellt werden, ob es sich um ein Abutment oder eine Vollprothese handelt.

Jeder Punkt in den Diagrammen stellt einen einzelnen Klassifikationsdatensatz 101 für ein digitales Dentalobjekt 100-1 als Eingabedaten dar. Bei einer Nächste-Nachbarn-Klassifikation wird eine nichtparametrische Methode zur Schätzung von Wahrscheinlichkeitsdichtefunktionen für die Klassifikationsdatensätze 101 durchgeführt. Der daraus resultierende K-Nearest-Neighbor-Algorithmus ist ein Klassifikationsverfahren, bei dem eine Klassenzuordnung unter Berücksichtigung seiner k nächsten Nachbarn vorgenommen wird.

Bei einer Klassifikation mit einer linearen Support Vector Machine - SVM (Stützvektormaschine) für die Klassifikationsdatensätze 101 wird ein Klassifikator und ein Regressor zur Regressionsanalyse verwendet. Die Support Vector Machine unterteilt die Menge von digitalen Dentalobjekten so in Klassen, dass um die Klassengrenzen herum ein möglichst breiter Bereich frei von Objekten bleibt. Die Support Vector Machine ist ein sogenannter Large Margin Classifier (Breiter-Rand-Klassifikator). Zusätzlich kann eine radiale Basisfunktion (RBF) verwendet werden, deren Wert vom Abstand zum Ursprung abhängig ist. Bei einer Klassifikation mit einen Gauß-Verfahren wird ein stochastischer Prozess für die Klassifikationsdatensätze 101 verwendet, bei dem jede endliche Teilmenge von Zufallsvariablen mehrdimensional normalverteilt (gaußverteilt) ist.

Jeder Klassifikationsdatensatz 101 kann durch das Klassifizierungsverfahren einer Klasse 103-1, ..., 103-n zugeordnet werden, wie beispielsweise, ob es sich um die Klasse "Abutment" oder die Klasse "Vollprothese" handelt. Das Herstellungsverfahren für ein reale Dentalobjekt 100-2 kann schließend auf Basis der Klasse des digitalen Dentalobjektes 100-1 bestimmt werden.

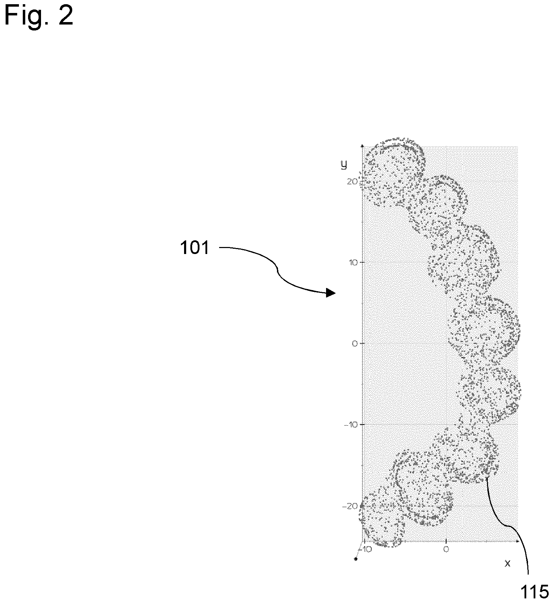

Fig. 2 zeigt eine Vielzahl von Punkten 115 auf der Oberfläche des digitalen Dentalobjektes 100-1, die in einem Klassifikationsdatensatz 101 vorhanden sein können. Die Punkte 115 sind jeweils durch die Koordinaten X, Y, und Z eines Koordinatensystems festgelegt.

Dieser Klassifikationsdatensatz 101 wird durch das Klassifikationsverfahren der Klasse "Vollprothese" zugeordnet. Um das Klassifikationsverfahren invariant gegenüber einer Eingabepermutation zu machen, werden symmetrische Funktionen verwendet. Eine symmetrische Funktion ist eine Funktion, bei der die Variablen untereinander vertauscht werden können, ohne den Funktionswert zu ändern.

Das Klassifikationsverfahren kann mit einer Vielzahl von Klassifikationsdatensätzen 101 angelernt werden, von denen die jeweilige Klasse bekannt ist. Neue Klassifikationsdatensätze 101 können dann auf Basis ihrer Ähnlichkeit zu den gelernten Klassifikationsdatensätzen 101 klassifiziert werden.

Bei der Klassifizierung lernt der Klassifizierungsalgorithmus beim Training je nach Eingabedaten und deren Eigenschaften selbstständig ein geeignetes Ähnlichkeitsmaß, um die verschiedenen Klassen von Dentalobjekten 100-1 zu unterscheiden. Daher ist es nicht erforderlich, ein explizites Ähnlichkeitsmaß zu definieren. Der Klassifizierungsalgorithmus bestimmt, wie beispielsweise ein neuronales Netz, während der Trainingsphase auf Basis der Trainingsdaten implizit selbst, welche Eigenschaften der Punktwolken relevant sind, um eine Ähnlichkeit abzubilden. Dies wird dann zur Klassifizierung des Klassifikationsdatensätze 101 verwendet. Je nach Form des Trainingsdatensatzes kann das Ähnlichkeitsmaß variieren. Hierbei handelt es sich um einen Effekt, der grundsätzlich bei komplexeren Klassifizierungsalgorithmen auftritt und der bewusst in Kauf genommen wird.

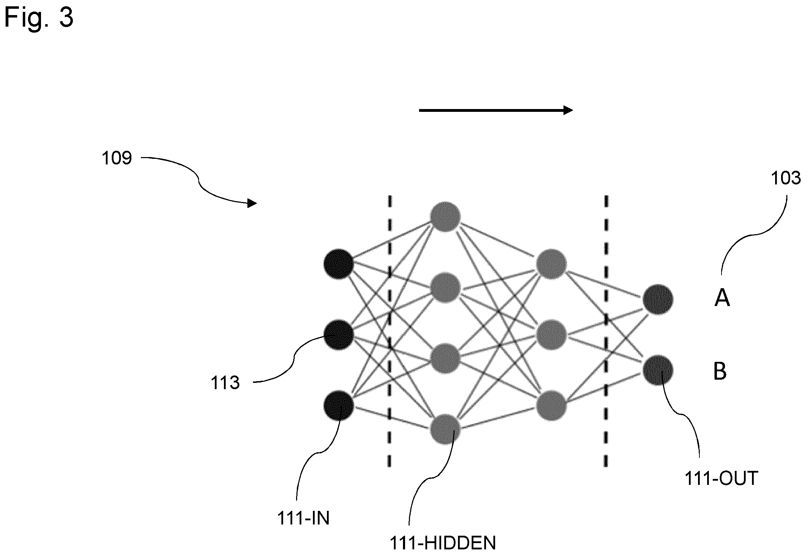

Fig. 3 zeigt eine schematische Ansicht eines künstlichen neuronalen Netzes 109. Das künstliche neuronale Netz 109 ist ein Netz aus künstlichen Neuronen und kann zum Zuordnen des digitalen Dentalobjektes 100-1 verwendet werden. Das künstliche neuronale Netz 109 umfasst eine Eingabeschicht 111-IN mit einer Anzahl an Neuronen 113, die beispielsweise der Anzahl der Punkte 115 auf der Oberfläche des digitalen Dentalobjektes 100-1 im Klassifikationsdatensatz 101 entspricht. In diesem Fall wird jeder Punkt aus dem Klassifikationsdatensatz 101 in ein eigenes Neuron 113 eingegeben.

Diese werden an die Neuronen 113 verborgener Schichten 111-Hidden weitergeleitet. Dabei findet eine individuelle Gewichtung jedes Signals von einem Neuron 113 zu einem anderen Neuron 113 statt. Anschließend wird das Ergebnis an der Ausgabeschicht 111-OUT ausgegeben. Die Anzahl der Neuronen 113 der Ausgabeschicht 111-OUT entspricht beispielsweise der Anzahl der Klassen. Auf diese Weise entsteht eine Abbildung:
F(X₁,Y₁,Z₁, ..., Xₙ,Yₙ,Zₙ) -> Klasse₁, ..., Klasseₘ

Bei einem Anlernen des neuronalen Netzes 109 wird eine Vielzahl von Klassifikationsdatensätzen 101 zugeführt, von denen die jeweilige Klasse bekannt ist. Das neuronale Netz 109 lernt durch eine Modifikation der Gewichte zwischen den Neuronen 113. Dabei werden die Gewichte so lange angepasst, bis die ausgegebene Klasse derjenigen Klasse entspricht, die für den Klassifikationsdatensatz 101 bekannt ist.

Im Allgemeinen kann eine Kombination aus Faltungsschichten (Convolutional Layer) und voll verbundenen Schichten verwendet werden (Dense Layer). Als Aktivierungsfunktionen können Sigmoid-, Tanh- oder ReLU-Funktionen verwendet werden. Nach jeder Schicht kann eine Batch-Normalisierung durchgeführt werden.

Die Erfindung lässt sich beispielsweise durch folgenden Quellcode ausführen:

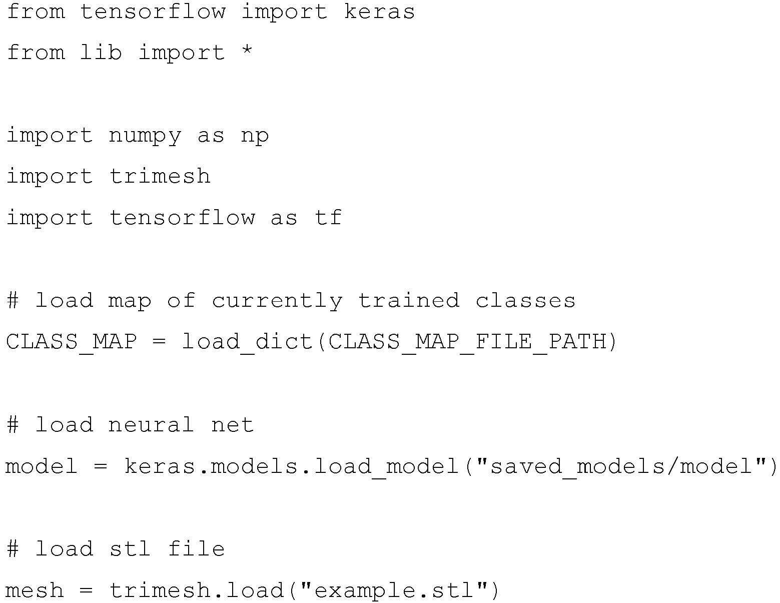

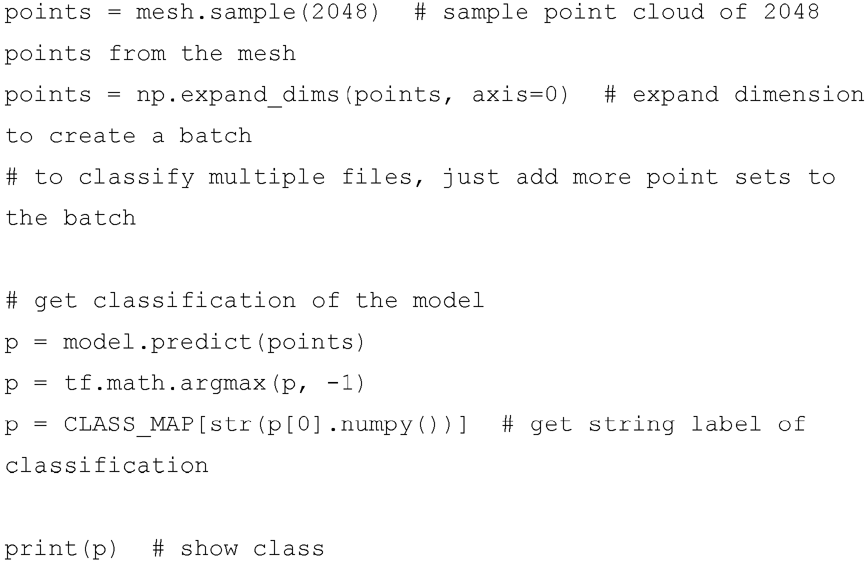

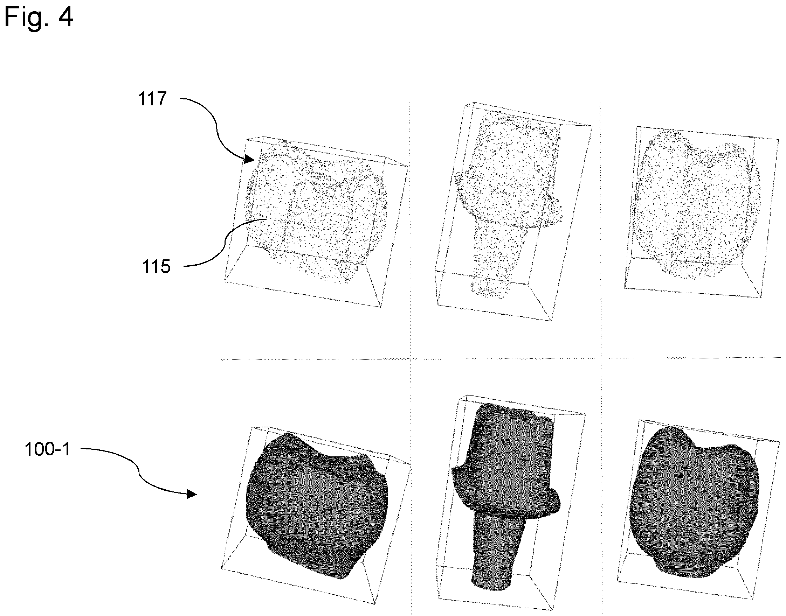

Fig. 4 zeigt mehrere digitale Dentalobjekte 100-1 unterschiedlicher Klassen, wie beispielsweise eine Krone (links), ein Abutment, (Mitte) und eine weitere Krone (rechts). Die Oberfläche dieser Dentalobjekte 100-1 wird durch eine Punktewolke 117 von Zufallspunkten beschrieben.

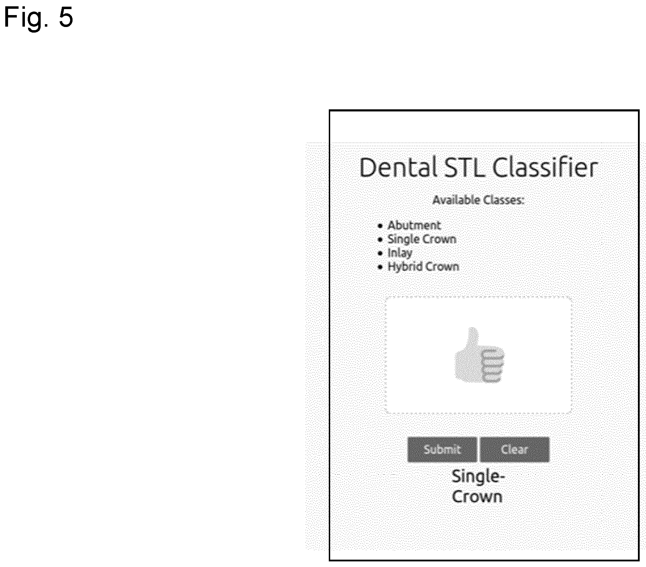

Fig. 5 zeigt eine Anzeige einer erkannten Klasse. Die Klasse des digitalen Dentalobjekts 100-1 kann angezeigt werden. Dann kann wiederum eine eindeutige Kennung zum Datensatz des digitalen Dentalobjekts 100-1 hinzugefügt werden. Dadurch kann ein zunächst unbekannter Datensatz zu einem Datensatz werden, der zutreffend klassifiziert ist und wieder für ein Anlernen des Klassifizierungsalgorithmus verwendet werden kann.

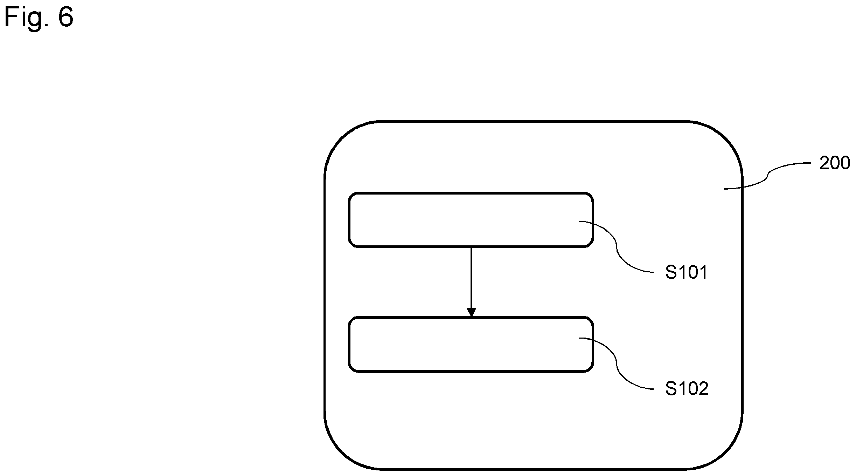

Fig. 6 zeigt ein Blockdiagramm eines Erkennungsverfahren für ein Dentalobjekt 100-1. Das Erkennungsverfahren umfasst den Schritt S101 eines Bereitstellens des digitalen Dentalobjekts 100-1 in einem Koordinatensystem, das eine Form des herzustellenden Dentalobjekts 100-2 beschreibt. Hierzu wird beispielsweise der dentale 3D-Datensatz des digitalen Dentalobjektes 100-1 importiert. Danach kann eine Vielzahl von gleichmäßig verteilten Zufallspunkten auf der Oberfläche des Dentalobjektes 100-1 ermittelt werden, wie beispielsweise 4096 Zufallspunkte und in einem Klassifikationsdatensatz 101 gespeichert werden.

Anschließend wird In Schritt S102 das digitale Dentalobjekt 100-1 zu einer vorgegebenen Klasse auf Basis der Form durch einen selbstlernenden Algorithmus automatisch zugeordnet. Hierzu wurde dieser Algorithmus zuvor mit einer Vielzahl von bekannten digitalen Dentalobjekten 100-1 und deren jeweiliger dentalen Klassifikation und/oder Indikation angelernt. Jede Indikation ist die eine Teilmenge einer Klasse. Der selbstlernende Algorithmus kann die Parameter einer Funktion optimieren, die die geometrischen Informationen des digitalen Dentalobjekts 100-1 auf die jeweilige Klasse abbildet.

Durch das Erkennungsverfahren kann die Identifikation der dreidimensionalen Datensätze für die digitalen Dentalobjekte 100-1 beim Import in die CAM-Software automatisch und ohne angehängte Metadaten erfolgen. Dabei wird kein statischer Algorithmus verwendet, um bestimmte Merkmale des digitalen Dentalobjektes 100-1 explizit zu identifizieren, sondern ein dynamischer Machine-Learning-Algorithmus, von dem die Merkmale implizit gelernt werden.

Das Ergebnis ist abhängig von der dreidimensionalen Struktur und der Anzahl der zur Verfügung stehenden digitalen Dentalobjekte 100-1 mit einer Indikation, die zum Anlernen verwendet worden sind. Danach kann in einem weiteren Schritt eine Überprüfung und Plausibilitätsprüfung der Klassifizierung anhand von einfachen geometrischen Merkmalen durchgeführt werden, wie beispielsweise auf Basis von Abmessungen des digitalen Dentalobjekts 100-1 (Bounding Box, Zentroid, lokale Krümmung).

Die Plausibilitätsprüfung wird nach der Klassifizierung durchgeführt, wenn das Klassifikationsergebnis bereits feststeht, um dieses nochmals zu überprüfen. Zur Prüfung der Plausibilität kann zum Beispiel das Volumen des Dentalobjekts 100-1 verwendet werden. Anhand des Volumens kann beispielsweise geprüft werden, ob das vorherige Klassifikationsergebnis "Einzelkrone" zutreffend ist. Anhand des Volumens kann überprüft werden, ob es sich tatsächlich um eine Einzelkrone handelt oder ob das Volumen für eine Einzelkrone zu groß ist und eher dem Volumen einer Brücke entspricht.

Des Weiteren können für die Plausibilitätsprüfung Merkmale, wie zum Beispiel die Abmessungen eines Begrenzungsrahmens (Bounding-Box), eine maximale Größe der Bounding Box, die Seitenverhältnisse der Bounding Box, eine Krümmung des Dentalobjektes oder eine Position des Masseschwerpunktes des Dentalobjektes verwendet werden. Hierzu können auch Kombinationen von mehreren Merkmalen verwendet werden.

Um die optimale Orientierung und Positionierung des digitalen Dentalobjektes 100-1 im zahntechnischen Arbeitsablauf durch die CAM-AM-Software zu ermöglichen, sollte eine Klassifikation des digitalen Dentalobjekts 100-1 bekannt sein. In diesem Fall können beispielsweise Regeln für eine Orientierung des digitalen Dentalobjekt festgelegt sein, so dass automatisch eine optimierte Stützstrukturerzeugung durchgeführt werden kann.

Ist die Klasse, d.h. die Indikation, des digitalen Dentalobjektes 100-1 der CAD-CAM-Software bekannt, können auf dieser Basis weitere Maßnahmen durchgeführt werden, die den Arbeitsablauf bei der Herstellung optimieren. Diese Maßnahmen beinhalten beispielsweise die Optimierung einer Ausrichtung des digitalen Dentalobjektes dahingehend, dass hergestellte Dentalobjekt möglichst wenig Stützstrukturen benötigt und ein Großteil der Oberfläche des Dentalobjektes nicht mehr nachbearbeitet werden muss.

Hierzu kann aus einer Datenbank beispielsweise ein digitales Referenzobjekt auf Basis der ermittelten Klasse abgerufen werden. Würde beispielsweise erkannt, dass es sich bei dem digitalen Dentalobjekt 100-1 um eine Vollprothese handelt, kann aus der Datenbank ein digitales Referenzobjekt der Klasse "Vollprothese" abgerufen werden. Das digitale Dentalobjekt 100-1 kann anschließend an das Referenzobjekts im Koordinatensystem transformiert werden. Beispielsweise wird eine Orientierung, eine Position oder eine Größe des digitalen Dentalobjekts 100-1 an das Referenzobjekt angepasst. Zudem kann für das Referenzobjekt der jeweiligen klasse ein Herstellungsverfahren vorgesehen sein, das für die Herstellung des digitalen Dentalobjekts verwendet wird.

Das Erkennungsverfahren für das Dentalobjekt 100-1 kann in einem Herstellungsgerät 200 implementiert sein, mit dem sich die realen Dentalobjekte 100-2 auf Basis der digitalen Dentalobjekte 100-1 herstellen lassen, wie beispielsweise einer Druckvorrichtung für einen 3D-Druck oder einer Fräsvorrichtung. Zu diesem Zweck umfasst das Herstellungsgerät 200 beispielsweise einen Computer mit einem digitalen Speicher und einem Prozessor zum Ausführen eines Computerprogramms, durch das das Erkennungsverfahren implementiert wird.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100-1: digitales Dentalobjekt
- 100-2: herzustellendes Dentalobjekt
- 101: Klassifikationsdatensatz
- 103: Klasse
- 109: künstliches neuronales Netz
- 111: Schicht
- 113: Neuron
- 115: Punkt
- 117: Punktewolke

- 200: Herstellungsgerät

## Patentansprüche

1. Erkennungsverfahren für ein Dentalobjekt (100-1), mit den Schritten:
- Bereitstellen (S101) eines digitalen Dentalobjekts (100-1) in einem Koordinatensystem, das eine Form des herzustellenden Dentalobjekts (100-2) beschreibt; und
- Automatisches Zuordnen (S102) des digitalen Dentalobjektes (100-1) zu einer vorgegebenen Klasse auf Basis der Form durch einen selbstlernenden Algorithmus;
- Zuordnen eines Herstellungsverfahrens zu dem digitalen Dentalobjekt (100-1) auf Basis der zugeordneten Klasse; und
- Hinzufügen weiterer räumlicher Strukturen zu dem digitalen Dentalobjekt (100-1) auf Basis des zugeordneten Herstellungsverfahrens.

2. Erkennungsverfahren nach Anspruch 1, wobei eine Anzahl von Punkten auf der Oberfläche des digitalen Dentalobjekts (100-1) erfasst wird.

3. Erkennungsverfahren nach Anspruch 2, wobei die Punkte auf der Oberfläche des digitalen Dentalobjekts (100-1) zufällig ausgewählt werden.

4. Erkennungsverfahren nach Anspruch 2 oder 3, wobei die Koordinaten der erfassten Punkte eine Eingabe für ein künstliches neuronales Netz (109) bilden.

5. Erkennungsverfahren nach einem der Ansprüche 2 bis 4, wobei das künstliche neuronales Netz (109) durch eine Mehrzahl von Trainingsdatensätzen angelernt worden ist.

6. Erkennungsverfahren einem der Ansprüche 2 bis 5, wobei die Klasse durch das künstliche neuronale Netz (109) ausgegeben wird.

7. Erkennungsverfahren nach einem der vorangehenden Ansprüche, wobei dem digitalen Dentalobjekt (100-1) auf Basis der zugeordneten Klasse ein digitales Referenzobjekt zugeordnet wird.

8. Erkennungsverfahren nach einem der vorangehenden Ansprüche, wobei das digitale Dentalobjekt (100-1) auf Basis der zugeordneten Klasse und/oder des Referenzobjekts im Koordinatensystem transformiert wird.

9. Erkennungsverfahren nach Anspruch 1, wobei das Dentalobjekt (100-2) durch das Herstellungsverfahren hergestellt wird.

10. Erkennungsverfahren nach Anspruch 9, wobei das Herstellungsverfahren ein additives oder subtraktives Herstellungsverfahren ist.

11. Herstellungsgerät (200) für ein Dentalobjekt (100-2), das ausgebildet ist, das Erkennungsverfahren nach einem der Ansprüche 1 bis 10 durchzuführen.

12. Computerprogramm, mit Befehlen, die bei der Ausführung des Computerprogramms durch einen Computer diesen veranlassen, das Verfahren nach einem der Ansprüche 1 bis 8 auszuführen.

## Claims

1. A recognition method for a dental object (100-1), comprising the steps of:
- providing (S101) a digital dental object (100-1) in a coordinate system describing a shape of the dental object (100-2) to be manufactured; and
- automatically assigning (S102) the digital dental object (100-1) to a specified class based on the shape by a self-learning algorithm;
- assigning a manufacturing method to the digital dental object (100-1) based on the assigned class; and
- adding further spatial structures to the digital dental object (100-1) based on the assigned manufacturing method.

2. The recognition method according to claim 1, wherein a number of points on the surface of the digital dental object (100-1) is detected.

3. The recognition method according to claim 2, wherein the points on the surface of the digital dental object (100-1) are randomly selected.

4. The recognition method according to claim 2 or 3, wherein the coordinates of the detected points form an input for an artificial neural network (109).

5. The recognition method according to any one of claims 2 to 4, wherein the artificial neural network (109) has been trained by a plurality of training data sets.

6. The recognition method according to any one of claims 2 to 5, wherein the class is output by the artificial neural network (109).

7. The recognition method according to any one of the preceding claims, wherein a digital reference object is assigned to the digital dental object (100-1) based on the assigned class.

8. The recognition method according to any one of the preceding claims, wherein the digital dental object (100-1) is transformed based on the assigned class and/or the reference object in the coordinate system.

9. The recognition method according to claim 1, wherein the dental object (100-2) is manufactured by the manufacturing method.

10. The recognition method according to claim 9, wherein the manufacturing method is an additive or subtractive manufacturing method.

11. A manufacturing apparatus (200) for a dental object (100-2), which is configured to perform the recognition method according to any one of claims 1 to 10.

12. A computer program, comprising instructions which, when the computer program is executed by a computer, cause the computer to execute the method according to any one of claims 1 to 8.

## Revendications

1. Procédé de reconnaissance pour un objet dentaire (100-1), comprenant les étapes suivantes :
- la fourniture (S101) d'un objet dentaire numérique (100-1) dans un système de coordonnées décrivant une forme de l'objet dentaire à fabriquer (100-2) ; et
- l'assignation automatique (S102) de l'objet dentaire numérique (100-1) à une classe prédéterminée en fonction de la forme par un algorithme d'auto-apprentissage ;
- l'assignation d'un processus de fabrication à l'objet dentaire numérique (100-1) en fonction de la classe attribuée ; et
- l'ajout de structures spatiales supplémentaires à l'objet dentaire numérique (100-1) en fonction du processus de fabrication associé.

2. Procédé de reconnaissance selon la revendication 1, où un certain nombre de points sur la surface de l'objet dentaire numérique (100-1) est détecté.

3. Procédé de reconnaissance selon la revendication 2, où les points à la surface de l'objet dentaire numérique (100-1) sont sélectionnés au hasard.

4. Procédé de reconnaissance selon la revendication 2 ou 3, où les coordonnées des points détectés constituent une entrée pour un réseau neuronal artificiel (109).

5. Procédé de reconnaissance selon l'une des revendications 2 à 4, où le réseau neuronal artificiel (109) a été formé à l'aide d'une pluralité d'ensembles de données d'apprentissage.

6. Procédé de reconnaissance selon l'une des revendications 2 à 5, où la classe est produite par le réseau neuronal artificiel (109).

7. Procédé de reconnaissance selon l'une des revendications précédentes, où un objet de référence numérique est attribué à l'objet dentaire numérique (100-1) sur la base de la classe attribuée.

8. Procédé de reconnaissance selon l'une des revendications précédentes, où l'objet dentaire numérique (100-1) est transformé dans le système de coordonnées sur la base de la classe attribuée et/ou de l'objet de référence.

9. Procédé de reconnaissance selon la revendication 1, où l'objet dentaire (100-2) est fabriqué par le procédé de fabrication.

10. Procédé de reconnaissance selon la revendication 9, où le procédé de fabrication est un procédé de préparation additif ou soustractif.

11. Appareil de fabrication (200) d'un objet dentaire (100-2) qui est configuré pour exécuter le procédé de reconnaissance selon l'une des revendications 1 à 10.

12. Programme informatique comprenant des commandes qui, lorsqu'elles sont exécutées par un ordinateur, amènent le programme informatique à mettre en œuvre le procédé selon l'une des revendications 1 à 8.
